# EUROPEAN PATENT APPLICATION

(11) **EP 3 871 604 A1**
(43) Date of publication of application: **01.09.2021**
(21) Application number: 21158515.3
(22) Date of filing: 22.02.2021
(51) Int. Cl.: A61B 6/00

(54) **RETRACTABLE DETECTOR ARM FOR INTERVENTIONAL C-ARM IMAGING SYSTEMS**

(30) Priority: 26.02.2020 US 202016801660
(71) Applicant: GE Precision Healthcare LLC, Wauwatosa, WI 53226 (US)
(72) Inventor: YESUDHAS, Jabez Augustine, Wauwatosa, WI 53226 (US)
(74) Representative: Fennell, Gareth Charles

(57) **Abstract**

An imaging gantry is provided that includes a C-arm having a detector mounted within the diameter of the C-arm at one end, and an X-ray tube mounted to the opposite end of the C-arm. The detector includes a retractable detector arm that is pivotally mounted to the C-arm at one end and has the detector mounted to the retractable detector arm opposite the C-arm. The detector is secured to a support plate that is attached to the planetary drive mechanism on the detector arm, such that the support plate swivels with the planetary drive mechanism to maintain the alignment of the support plate and detector relative to the X-ray tube. The detector arm also includes a lateral compensation mechanism disposed on the detector arm and engaged with the detector that operates in synchronization with the motor pivoting the detector arm to laterally displace the detector in order to maintain the alignment of the detector with the X-ray tube.

## Description

### BACKGROUND OF DISCLOSURE

The field of the invention relates to medical imaging generally, and more particularly, to interventional imaging systems.

Mobile or floor-mounted (fixed) X-ray devices are known that comprise moving parts enabling them to turn in different directions around a patient. These moving parts thereby enable a beam of X-rays to be oriented, so as to analyze a given part of the body of the patient, such as during an interventional procedure being performed on the patient. The images provided by the X-ray or imaging device enable the treating physician to view the area of the patient being treated in real-time during the procedure.

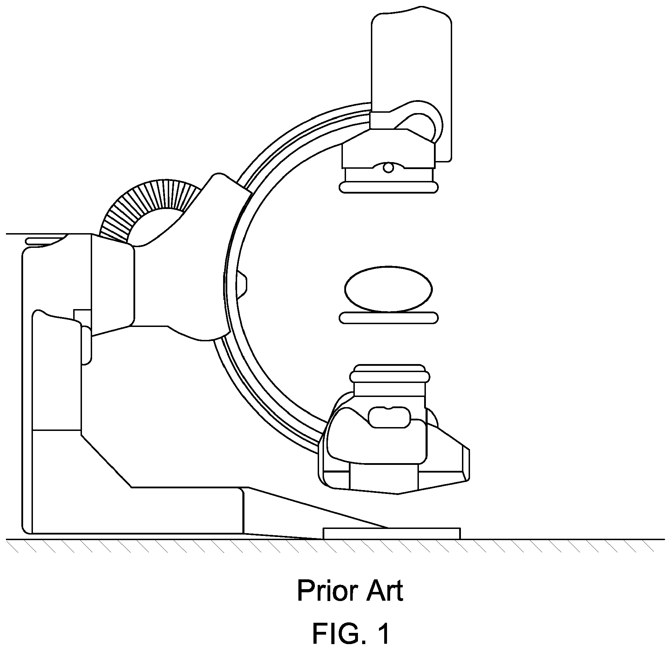

In these imaging systems, the imaging components of the gantry include an L-arm movably supporting the system on/over the floor, a pivot arm rotatably connected to the L-arm, and a C-arm movably connected to the pivot arm opposite the L-arm. The C-arm supports the X-ray tube at one end and the detector at the opposite end, in alignment with the X-ray tube. This configuration for the imaging system allows for the rotation of the gantry along three (3) axes around an isocenter, i.e., the point at which the rotation axes intersect and the point where the patient is located and supported on a patient bed during the imaging and interventional procedure, as shown in FIG. 1. The isocenter is located within the emission path of the X-rays from the tube towards the detector, such that the L-arm, pivot arm and C-arm are all moveable relative to the isocenter in order to achieve various imaging spins around the patient to obtain images used to reconstruct 3D volumes of the imaged areas of the patient.

However, with imaging monoplane gantries, while it is possible for the pivot arm to move the tube and detector on the C-arm, i.e., pivot angulation, around the patient (i.e., held at the isocenter by the patient bed) to produce images for 3D reconstruction in a frontal 3D spin, in which the tube and detector spin about pivot axis, the reach of the gantry allows for 3D imaging of only approximately the head and upper abdomen of the patient supported on the bed. The reach of the gantry is mainly limited by the diameter of the C-arc. In order to increase the reach, the C-Arm is offset from the isocenter, The pivot angulation is limited by the presence of this offset and the lift projection on the detector which extends beyond the height of the C-arm and which would contact the floor upon further pivot angulation.

In addition, while the gantry can move laterally along the length of the patient (with the patient held at the isocenter by the bed) in a lateral 3D spin, in which tube and detector spin about C-arc axis, the offset of the C-arm and the detector lift projection limit the movement of the C-arm relative to the pivot arm, i.e., the C-arc angulation, to approximately 95°. As a C-arc angulation of at least 180° is required for lateral 3D imaging, the gantry configuration with the detector lift projection and offset C-arm effectively prevents this.

Further, the protruding detector lift projection increases the chances of collision of the C-arm and lift projection with external objects, such as monitor suspensions within the theater in which the procedure is being performed. These collisions can result in sterility issues with the imaging gantry and part and/or paint or finish falling hazards.

In the prior art, many imaging gantries have been developed that increase the diameter of the C-arm in order to enable the detector lift projection to be better accommodated within the diameter of the C-arc. and thus increase the C-arc and pivot angulation However, this increase in the diameter of the C-arm raises the position of the isocenter for the gantry, necessitating an elevation in the patient bed when using these gantries, which creates issues for the physician performing the procedures.

Accordingly, it is desirable to develop an imaging gantry that includes a C-arm having a detector mounted thereto in manner that increases the C-arc angulation of the gantry to allow for the performance of lateral 3D spin angulation, without increase in diameter of C-arm.

### BRIEF DESCRIPTION OF THE DISCLOSURE

There is a need or desire for a imaging gantry, such as for interventional medical procedures, which includes a detector mounted thereon in a manner that allows for great movement of the detector around the patient or other object being imaged.

According to one exemplary non-limiting aspect of the disclosure, the gantry includes a C-arm having a detector mounted within the diameter of the C-arm at one end, and an X-ray tube mounted to the opposite end of the C-arm. The detector includes a retractable detector arm that is pivotally mounted to the C-arm at one end and has the detector mounted to the detector arm opposite the C-arm. The detector is secured to a support plate that is attached to the planetary drive mechanism on the detector arm, such that the support plate swivels with the planetary drive mechanism to maintain the alignment of the support plate and detector relative to the X-ray tube. The detector arm is moveable relative to the C-arm using a motor and a planetary belt or gear drive operably connected between the C-arm and the opposed end of the detector arm. The operation of the planetary belt drive pivots the detector arm relative to the C-arm to enable the detector to be positoned where desired.

According to another exemplary non-limiting embodiment of the disclosure, the detector arm includes a lateral compensation mechanism disposed on the detector arm and engaged with the detector. As the detector arm is pivoted relative to the C-arm by the motor, the lateral compensation mechanism laterally displaces the detector in order to maintain the alignment of the detector with the X-ray tube. The lateral compensation mechanism can be motorized or mechanical, and is operated in synchronization with the motor pivoting the detector arm relative to the C-arm.

According to still a further aspect of one exemplary non-limiting embodiment of the disclosure, the C-arm has the X-ray tube and the detector mounted within the diameter of the C-arm. Further, a C-arm guide used to secure the C-arm to the gantry can be positioned over the C-arm in a configuration that does not contact the detector arm, such that the C-arm can be moved greater than +/- 90° relative to the guide to allow for lateral 3D spin angulation.

According to still another aspect of one exemplary non-limiting embodiment of the disclosure, the positioning of the detector and optionally the X-ray tube within the diameter of the C-arm enables the gantry isocenter to be lowered with respect to prior art gantries that provide lateral 3D angulation, thereby enabling the patient to be positioned on the table at a comfortable height for the physician performing the procedure.

According to another aspect of one exemplary non-limiting embodiment of the disclosure, a detector positioning mechanism adapted to selectively position a detector in alignment with an X-ray tube on an imaging device includes a positioning arm adapted to be pivotally secured to the imaging device, a detector support plate pivotally connected to the positioning arm, and an arm movement mechanism engaged with the positioning arm to selectively move the positioning arm with regard to the imaging device.

According to still another aspect of one exemplary non-limiting embodiment of the disclosure, an imaging device includes a gantry, a C-arm movably connected to the gantry, an X-ray tube disposed at one end of the C-arm, a detector disposed on the C-arm opposite the X-ray tube, and a detector positioning mechanism connecting the detector to the C-arm, the detector positioning mechanism including a positioning arm pivotally secured to the C-arm, a detector support plate pivotally connected to the positioning arm opposite the C-arm, and an arm movement mechanism engaged between the C-arm and the positioning arm to selectively move the positioning arm with regard to the C-arm.

According to still another aspect of one exemplary non-limiting embodiment of the disclosure, a method of obtaining images of an object with an imaging device includes the steps of providing an imaging device including a gantry, a C-arm movably connected to the gantry, an X-ray tube disposed at one end of the C-arm, a detector disposed on the C-arm opposite the X-ray tube completely within a diameter of the C-arm and a detector positioning mechanism completely within a diameter of the C-arm connecting the detector to the C-arm, the detector positioning mechanism including a positioning arm pivotally secured to the C-arm, a detector support plate pivotally connected to the positioning arm opposite the C-arm, and an arm movement mechanism engaged between the C-arm and the positioning arm to selectively move the positioning arm with regard to the C-arm, moving the positioning arm to locate the detector where desired relative to the object, and taking an image of the object.

It should be understood that the brief description above is provided to introduce in simplified form a selection of concepts that are further described in the detailed description.

### BRIEF DESCRIPTION OF THE DRAWINGS

The drawings illustrate the best mode presently contemplated of carrying out the disclosure. In the drawings:
FIG. 1 is a side elevational view of a prior art interventional imaging gantry.
FIG. 2 is a side elevational view of an interventional imaging gantry according to one exemplary non-limiting embodiment of the invention with the detector in an extended position.
FIG. 3 is a side elevational view of the interventional imaging gantry of FIG. 2 with the detector in a retracted position.
FIG. 4 is an isometric view of detector arm of the gantry of FIG. 2.
FIG. 5 is a partially broken away, isometric view of the detector arm of the gantry of FIG. 2.
FIG. 6 is a side elevational view of the interventional imaging gantry of FIG. 2 including a protective bellows.
FIG. 7 is a partially broken away, isometric view of the detector arm of FIG. 5 with a first embodiment of a lateral compensation mechanism.
FIG. 8 is a partially broken away, isometric view of the detector arm of FIG. 5 with a second embodiment of a lateral compensation mechanism.
FIG. 9 is a partially broken away, isometric view of the detector arm of FIG. 8.
FIG. 10 is a side elevational view of the gantry of FIG. 2 at one end of a lateral 3D spin configuration.
FIG. 11 is a side elevational view of the gantry of FIG. 2 at an opposite end of a lateral 3D spin configuration.
FIG. 12 is a schematic view of an interventional imaging gantry according to one exemplary non-limiting embodiment of the invention with offset brackets for mounting the X-ray tube and detector to the gantry.

### DETAILED DESCRIPTION

In the following detailed description, reference is made to the accompanying drawings that form a part hereof, and in which is shown by way of illustration specific embodiments, which may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken in a limiting sense.

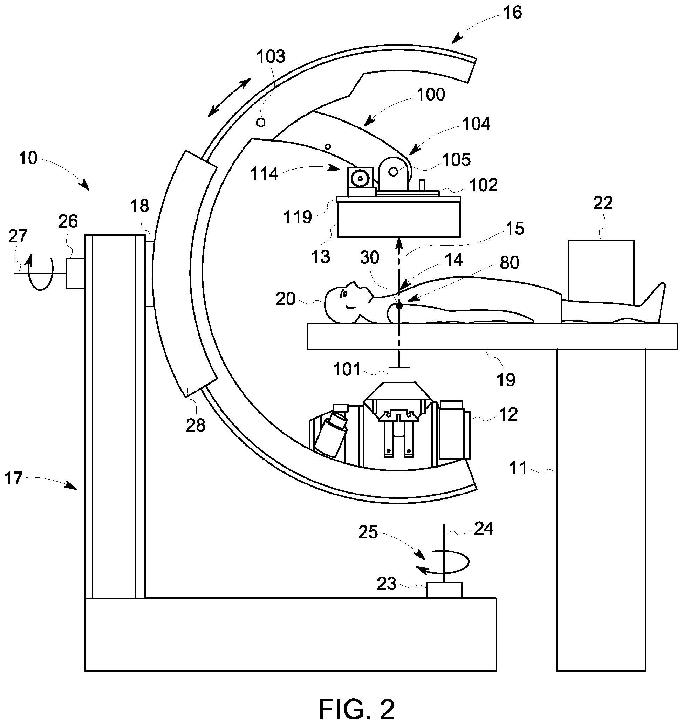

Referring to FIG. 2, in the illustrated exemplary non-limiting embodiment a medical imaging system or device 10, which may be fixed in place or movable, includes in particular an X-ray tube 12 and an X-ray detector 13. This tube 12 emits a beam 14 of X-rays along an emission direction 15. The tube 12 and the detector 13 are both coupled to the ends, on either side, of a C-shaped arm/C-arm/C-arc 16. Indeed, the detector 13 is coupled to the C-arm 16 opposite the tube 12 and in the emission direction 15, so as to receive the beam 14 of rays. Moreover, the arm 16 is connected to an L-shaped support arm 17 with the help of an intermediate pivot arm 18. A collimator 101 placed immediately above the tube 12 may enable a shape to be given to the beam 14 of X-rays emitted by the tube 12. Thus, this collimator 101 may in particular modify the width of the beam 14.

A bed 19 on which a patient 20 is laid out is coupled to a frame 11. This bed 19 is placed within the C-shaped arm 16, so that the tube 12 is located under the bed 19 and the detector 13 above the bed 19. Whatever the examination performed, the tube 12 and the detector 13 preferably always maintain this spatial configuration. The tube 12 may in certain examinations be located above the examination table or bed 19 and the detector 13 underneath the examination table or bed 19.

Under these conditions, after having received the beam 14 that passes through a part of the body of the patient, the detector 13 emits electrical signals corresponding to the intensity of the rays received. These electrical signals may then be transmitted to a computer 22 via wire connections not shown. These electrical signals can enable this computer 22 to produce an image corresponding to the part of the body analyzed. This image may be viewed by means of a screen of this computer 22 within the context of radioscopy, or printed out on a sheet within the context of radiography.

With the aim of being able to examine each part of the body of the patient 20, the beam 14 may be oriented in a multitude of directions around the patient. Indeed, the position of the tube 12 and the detector 13 may be modified by a user. To this end, the L-shaped support arm 17, the intermediate pivot arm 18 and the C-shaped arm 16 forming parts of the gantry or device 10 are all hinged in relation to each other. More precisely, the L-shaped support arm 17 is hinged around or movable with respect to the ground relative to the frame 11 through the intermediary of a first motor 23. This motor 23 thereby enables the strut 17 to rotate around a vertical axis 24 in a rotation direction 25.

The pivot arm 18 can rotate with respect to the support arm 17 around a horizontal axis 27 defined by the center of the connection point of the pivot arm 18 to the support arm 17. The pivot arm 18 can move bout this axis by operation of a similar motor 26 operably connected to the pivot arm 18 within the support arm 17.

As regards the C-shaped arm/C-arm 16, it can slide with respect to a link 28 that is disposed around and engaged with the exterior of the C-arm 16. The C-arm 16 may thereby turn around an axis 30 extending perpendicularly into and out of the page of FIG. 2 that passes through the center of a circle formed by two C-arms 16 placed side by side. This axis 30 is moreover perpendicular to both the axis 27 and the axis 24 for the position represented, with each axis 24, 27 and 30 extending through the isocenter 80 of the gantry or device 10.

By combining the rotational movements around the three axes 24, 27 and 30, the beam 14 of X-rays can describe all the emission directions of the X-rays included within a sphere. Thanks to the motors 23 and 26 and the motor that drives link 28, the beam 14 can thereby pass through each part of the patient along a multitude of possible orientations.

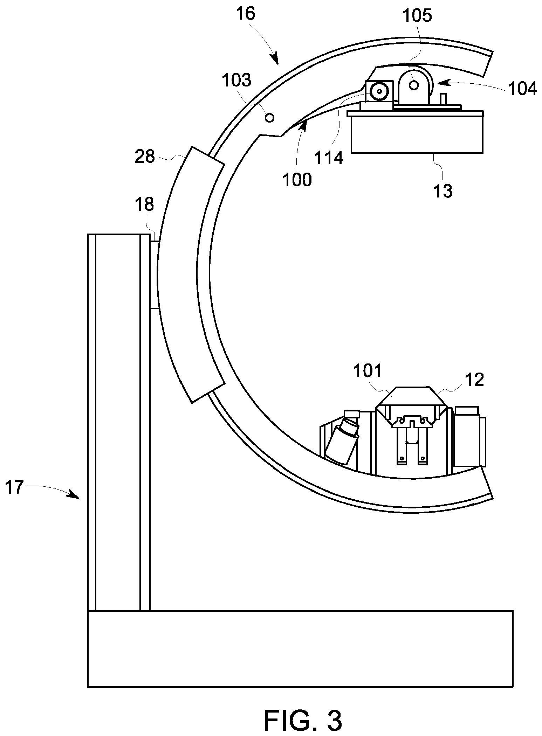

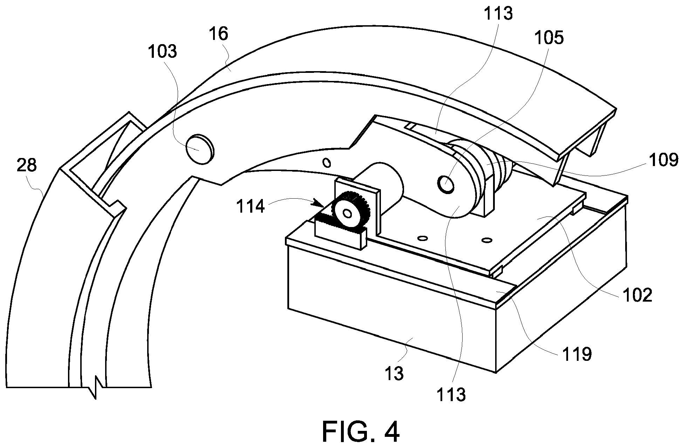

Referring now to FIGS. 2-5, the detector 13 is connected to a positioning mechanism 90 including a positioning arm 100 that is pivotally attached to the C-arm 16 within the diameter of the C-arm 16, and in the illustrated exemplary embodiment within opposed sides of the C-arm 16. The positioning arm 100 is secured at one end to the C-arm 16 via a hinge or pivot pin 103 that is movably secured to the C-arm 16 and to which the positioning arm 100 is fixed. In a fully retracted position (see FIG. 3), the positing arm 100 is completely housed within the C-arm 16, and at least partially disposed between opposed sides of the C-arm 16. The pivot pin 103 is movably/rotatably secured to the C-arm 16 to enable the positioning arm 100 to rotate with the pivot pin 103 when the pivot pin 103 is rotated by a motor 112 connected to the pivot pin 103.

Generally opposite the pivot pin 103, the positioning arm 100 is connected to a detector support plate 102 via a hinge or pivot pin 105. The pivot pin 105 is disposed within an aperture 107 of a flange 109 extending outwardly from the support plate 102 and is fixed to the positioning arm 100 in order to enable the flange 109 and thus the support plate 102 to rotate with respect the pivot pin 105.

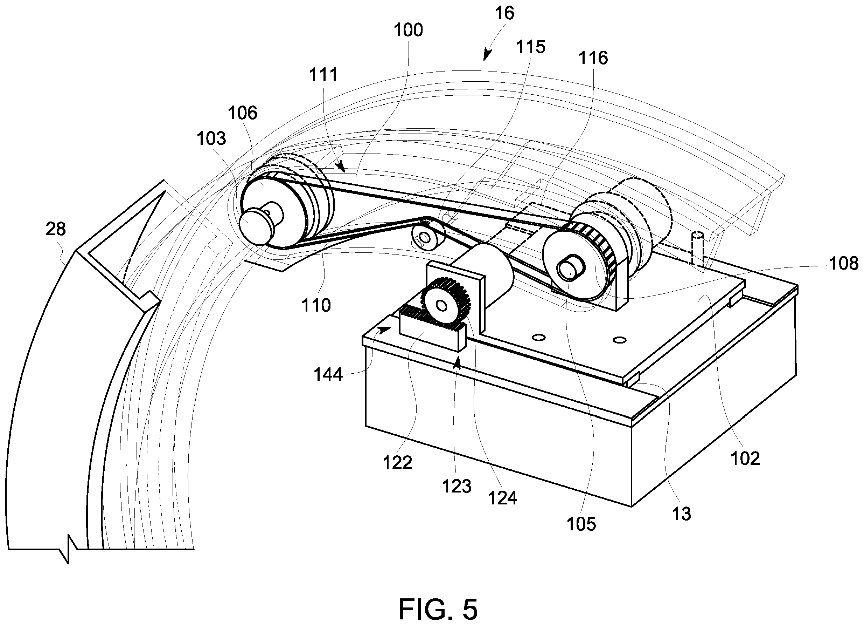

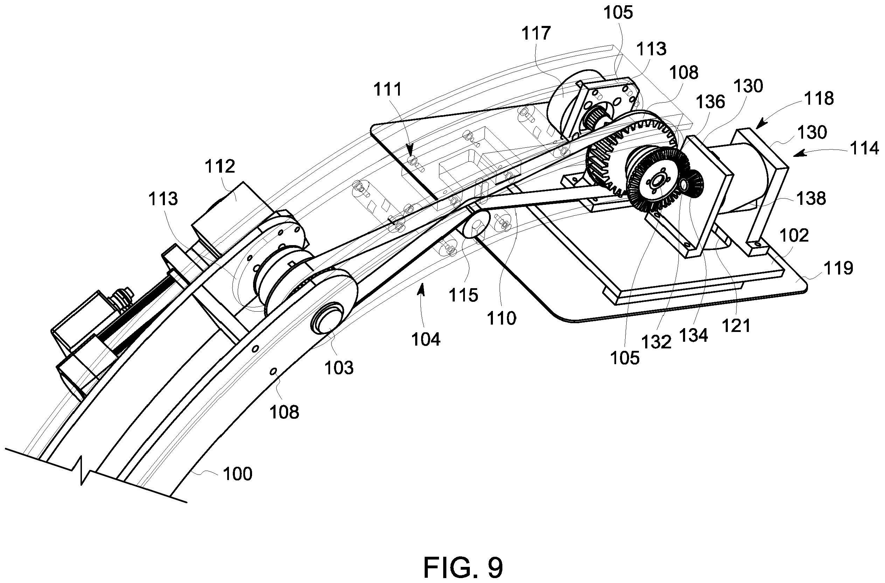

The positioning arm 100 includes an arm movement mechanism 111, which in the exemplary embodiment illustrated in FIG. 5 is planetary gear/belt system 104 having a stationary gear/pulley 106 secured to the C-arm 16 and disposed around the pivot pin 103 and a rotatable gear/pulley 108 disposed around the pivot pin 105 and fixed to the flange 109 on the support plate 102 that are joined by a belt 110. As shown in the exemplary embodiment of FIG. 9, the pulleys 106,108 can be disposed around the respective pivot pins 103,105 between opposed pairs of flanges or tabs 113 formed at each end of the positioning arm 100 and between which the pivot pins 103, 105 extend in order to reduce the overall length of the positioning mechanism 90 on the C-arm 16. Alternatively, the arm movement mechanism 111 be formed with other suitable drive mechanisms or trains, such as a bevel gear train (not shown), among others. The arm movement mechanism 111 can also include a number of idler pulleys 115 disposed on or within the positioning arm 100 between the stationary pulley 106 and the rotatable pulley 108 in order to provide additional tension on the belt 110, and a brake 117 engaged with the pivot pin 105 to prevent movement of the support plate 102 in the situation where the belt 110 malfunctions.

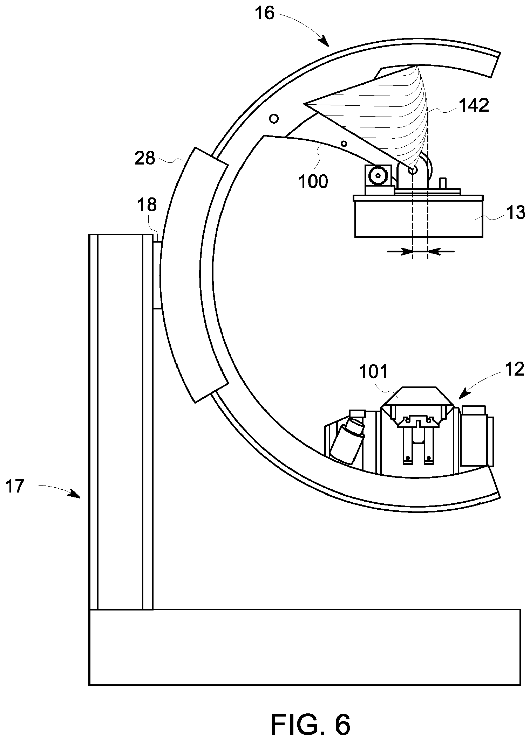

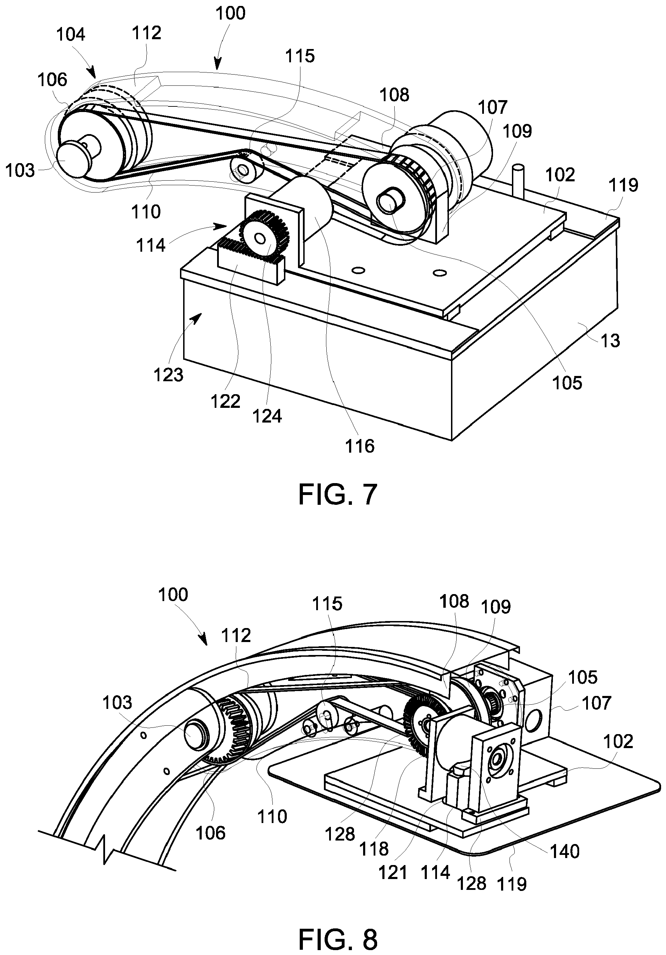

The system 104 is operated by the motor 112, such as a high torque, low RPM harmonic drive motor, that can be mounted to the positioning arm 100 (FIG. 8) or the C-arm 16 (FIG. 9). In the exemplary embodiment where the motor 112 is disposed on the C-arm 16, the motor 112 has its drive shaft (not shown) connected to or formed by the pivot pin 103. When the motor 112 is operated, the motor 112 rotates the pivot pin 103 and the arm 100 connected to the pivot pin 103 relative to the C-arm 16 along curve A shown in FIG. 6. In an alternative embodiment, a flexible bellows 142 is disposed between the positioning arm 100 and the C-arm 16 that can extend and retract in conjunction with the movement of the positioning arm 100 to prevent objects from inadvertently being positioned and/or pinched or caught between the positing arm 100 and the C-arm 16.

As the positioning arm 100 is rotated by the motor 112, the belt 110 moves around the stationary pulley 106 fixed to the C-arm 16 in a planetary movement manner. Concurrently, the belt 110 causes the rotating pulley 108 to rotate around the pivot pin 105. The rotation of the arm 100 causes the rotatable pulley 108 to rotate in the direction opposite to the direction of the rotation of the positioning arm 100, with the belt 110 moving along with the rotating pulley 108 to maintain the angle and/or orientation of the support plate 102, and the detector 13 attached thereto, relative to the tube 12. In this manner, the operation of the motor 112 to rotate the positioning arm 100 also causes the support plate 102 to move in conjunction with the rotatable pulley 108, as a result of the connection of the pulley 108 to the flange 109 on the support plate 102, such that the orientation of the support plate 102/detector 13 relative to the tube 12 is maintained throughout the range of movement of the positioning arm 100.

Further, as shown in FIGS. 6-9, the support plate 102 supports a lateral compensation mechanism 114 thereon to maintain the alignment of the detector 13 with the tube 12 and collimator 101. The mechanism 114 is disposed on the support plate 102 and is operably connected through an aperture 121 in the support plate 102 to a detector mounting plate 119 disposed beneath the support plate 102. The detector mounting plate 119 can be slidably coupled to the support plate 102 for lateral movement with regard to the support plate 102 under the operation of the compensation mechanism 114, and/or can be secured directly to the compensation mechanism 114. The lateral compensation mechanism 114 is engaged with the rotating pulley 108 to convert the rotation of the rotating pulley 108 into lateral movement of the detector mounting plate 119 that can slide laterally beneath the support plate 102. In this manner, the compensation mechanism 114 accommodates the lateral movement of the support plate 102 and detector 13 relative to the tube 12 during operation of the planetary belt system 104 to maintain the precise alignment of the detector 13 and the tube 12/collimator 101.

The movement of the compensation mechanism 114, which can be an electrically controlled compensation mechanism 123 including an electric motor 116 operated in concert with the motor 112 and engaged with a rack 122 and pinion 124 system (FIG. 7) in which the rack 122 is disposed on the mounting plate 119 and engaged with the pinion 124 located on the support plate 102 and rotated by the motor 116.

Alternatively, the mechanism 114 can take the form of a mechanical mechanism 118, including a cam 126 and follower 128 construction (FIGS. 8-9), that is synchronously operated with the planetary belt system 104 such that the operation of the planetary belt system 104 to move the positioning arm 100 causes the compensation mechanism 114 to correspondingly move the detector mounting plate 119 and the detector 13. In the illustrated exemplary embodiment of FIGS. 8 and 9, the cam 126 is rotatably mounted between brackets 130 on the support plate 102 and includes a shaft 132 that extends outwardly from between the brackets 130 to support a driven gear 134. The driven gear 134 is engaged with a drive gear 136 disposed on the adjacent end of the pivot pin 105, such that the drive gear 136 rotates along with the rotatable pulley 108. Rotation of the rotatable pulley 108 by operation of the motor 112 to move of the positioning arm 100 consequently rotates the pivot pin 105 and drive gear 136. The rotation of the drive gear 136 drives the driven gear 134 and shaft 132, causing the cam 126 on the shaft 132 to rotate between the brackets 130. The cam 126 includes a groove 138 formed therein in which is disposed a pin 140 of the follower 128 mounted to the mounting plate 119 and extending upwardly through the aperture 121 in the support plate 102. As the cam 126 rotates, the engagement of the pin 140 within the groove 138 causes the pin 140 to follow the shape/contour of the groove 138, thereby laterally moving the follower 138 and the mounting plate 119 relative to the support plate 102, providing the lateral compensation for the detector 13 relative to the tube 12. In alternative embodiments, multiple cams 136 and associated followers 138 can be utilized.

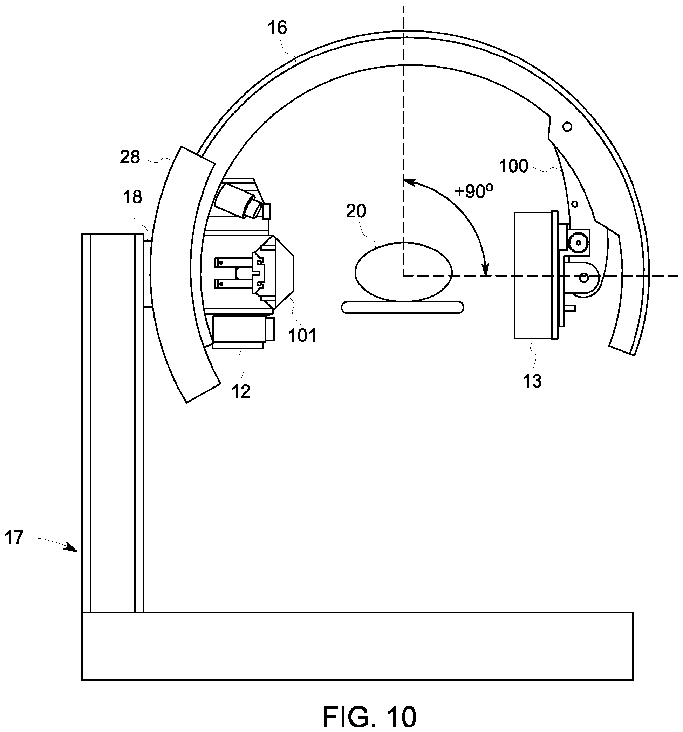

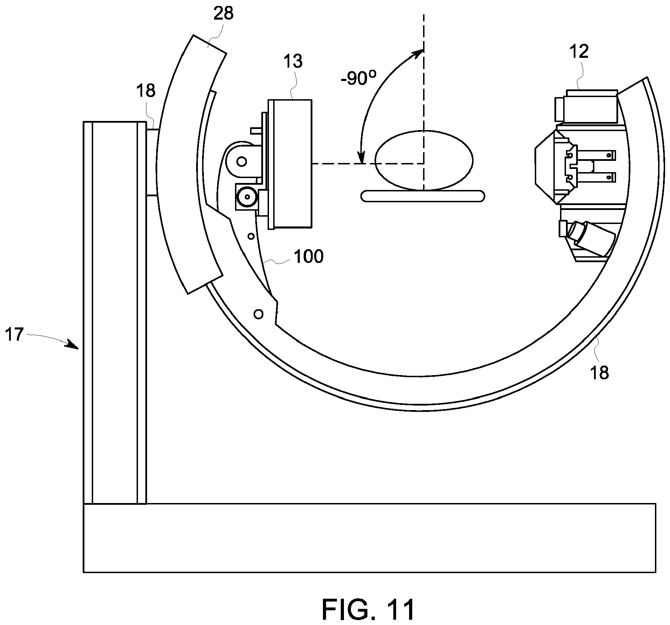

With this configuration of the detector 13 within the diameter of the C-arm 16 through the connection of the positioning arm 100, as shown in FIGS. 10-11, the C-arm 16 can be moved relative to the link 28 between positions of greater than +/- 90° relative to the position of the C-arm 16 in FIG. 2. Thus, the C-arm 16 including the detector 13 on the positioning arm 100 within the diameter of the C-arm 16 allows for a lateral 3D spin which requires at least a rotation of 180° relative to the isocenter.

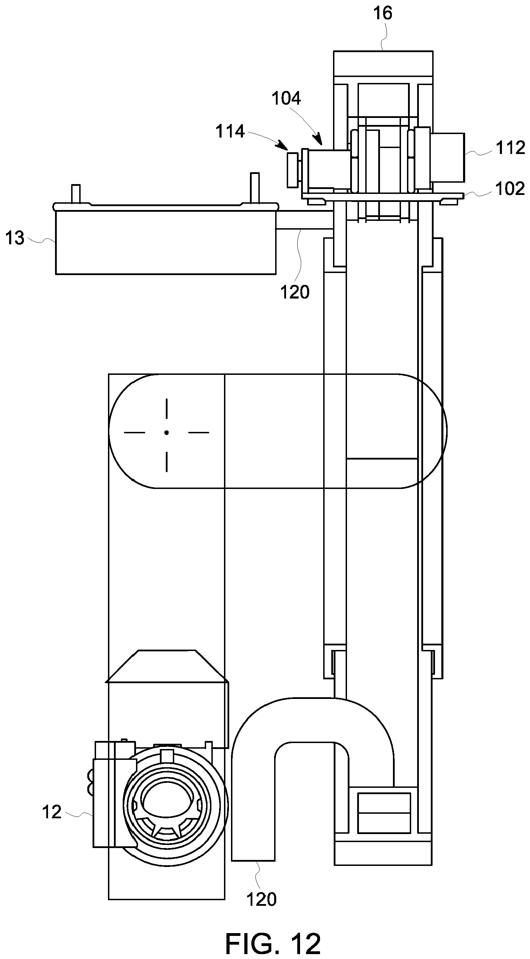

Other advantages provided by the construction of the device 10 including the detector positioning mechanism 90 disclosed herein include:
a. Enables Lateral 3D spin: Since there is no projection of detector 13 and/or detector lift/positioning mechanism 90 beyond the diameter of the C-arc 16, the C-arc guide/link 28 can go well over the detector lift/positioning mechanism 90 The support length of C-arc guide/link 28 can also be increased. The C-arc 16 movement can go >180 degrees with the same C-arc 16 diameter, when X-ray tube 12 is also housed completely within the C-arc diameter (see FIGS. 10-11), either by reduced size of tube 12 and collimator 101 or offset of tube 12 from C-arc 16 (as in FIG. 12 using offset brackets 120) - otherwise, can increase size of C-arc 16 to accommodate tube 12.
b. Low patient table working height as there is no increase in C-arc diameter - the proposed detector lift/positioning mechanism 90 enables lateral spin with the same existing C-arc diameter, thus height of isocenter from floor remain unchanged.
c. Enhanced frontal 3D - the existing frontal 3D spin angulation can also be enhanced as the detector lift/positioning mechanism 90 would not collide with L-arm base or floor during 3D spin. Enables better reconstructed image with lesser artifacts.
d. Lesser collision prone to external devices as there is no projection beyond C-arm/C-arc 16 - hence reduced sterility and part fall hazards in field.
e. Design can be implemented in frontal fixed plane, mobile gantry and in lateral plane - this design can also be considered in surgical C-arms.

The written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A detector positioning mechanism adapted to selectively position a detector in alignment with an X-ray tube on an imaging device, the detector positioning mechanism comprising:
- a positioning arm adapted to be pivotally secured to the imaging device;
- a detector support plate pivotally connected to the positioning arm; and
- an arm movement mechanism engaged with the positioning arm to selectively move the positioning arm with regard to the imaging device.

2. The detector positioning mechanism of claim 1 wherein the arm movement mechanism is a planetary belt drive.

3. The detector positioning mechanism of claim 2 wherein the planetary belt drive comprises:
- a stationary pulley disposed generally opposite the support plate and adapted to be secured to the imaging device;
- a rotating pulley secured to the support plate and rotatably attached to the positioning arm; and
- a belt connected between the stationary pulley and the rotating pulley.

4. The detector positioning mechanism of claim 3 wherein the planetary belt drive further includes a motor operably connected to the positioning arm and operable to rotate the positioning arm relative to the stationary pulley.

5. The detector positioning mechanism of claim 1 further comprising a lateral compensation mechanism operably connected between the arm movement mechanism and the support plate.

6. The detector positioning mechanism of claim 5 wherein the lateral compensation mechanism is an electrical lateral compensation mechanism.

7. The detector positioning mechanism of claim 5 wherein the lateral compensation mechanism is a mechanical lateral compensation mechanism.

8. An imaging device comprising:
- a gantry;
- a C-arm movably connected to the gantry;
- an X-ray tube disposed at one end of the C-arm;
- a detector disposed on the C-arm opposite the X-ray tube; and
- a detector positioning mechanism connecting the detector to the C-arm, the detector positioning mechanism comprising:
- a positioning arm pivotally secured to the C-arm;
- a detector support plate pivotally connected to the positioning arm opposite the C-arm; and
- an arm movement mechanism engaged between the C-arm and the positioning arm to selectively move the positioning arm with regard to the C-arm.

9. The imaging device of claim 8 further comprising a lateral compensation mechanism operably connected between the arm movement mechanism and the detector support plate.

10. The imaging device of claim 9 wherein the lateral compensation mechanism comprises:
- a cam disposed on the detector support plate and engaged with the arm movement mechanism;
- a detector mounting plate slidably secured to the detector support plate;
- a follower disposed on the detector mounting plate and engaged with the cam.

11. The imaging device of claim 8 wherein the arm movement mechanism is a planetary belt drive.

12. The imaging device of claim 8 wherein the positioning arm is retractable within the C-arm.

13. The imaging device of claim 8 wherein the detector is disposed completely within a diameter of the C-arm.

14. The imaging device of claim 8 wherein the tube is disposed completely within a diameter of the C-arm.

15. A method of obtaining images of an object with an imaging device, the method comprising the steps of:
- providing an imaging device including a gantry, a C-arm movably connected to the gantry, an X-ray tube disposed at one end of the C-arm, a detector disposed on the C-arm opposite the X-ray tube completely within a diameter of the C-arm and a detector positioning mechanism connecting the detector to the C-arm, the detector positioning mechanism comprising:
- a positioning arm pivotally secured to the C-arm;
- a detector support plate pivotally connected to the positioning arm opposite the C-arm; and
- an arm movement mechanism engaged between the C-arm and the positioning arm to selectively move the positioning arm with regard to the C-arm;
- moving the positioning arm to locate the detector where desired relative to the object; and
- taking an image of the object.
